(19) Europäisches Patentamt
European Patent Office
Office européen des brevets

(11) Publication number: 0 211 121 B1

(12) # EUROPEAN PATENT SPECIFICATION

(45) Date of publication of patent specification: 11.12.91 (51) Int. Cl.[5]: C07D 251/18

(21) Application number: 85305954.1

(22) Date of filing: 21.08.85

(54) **Process for synthesizing 2-vinyl-4,6-diamino-s-triazine.**

(43) Date of publication of application:
25.02.87 Bulletin 87/09

(45) Publication of the grant of the patent:
11.12.91 Bulletin 91/50

(84) Designated Contracting States:
BE CH DE FR GB IT LI

(56) References cited:
DE-A- 2 026 400
FR-A- 1 563 255
US-A- 4 579 946

CHEMICAL ABSTRACTS, vol. 102, 1985, page 9, no. 221315h, Columbus, Ohio, US; & JP - A - 60 19 772 (SHIKOKU CHEMICALS CORP.) 31-01-1985

CHEMICAL ABSTRACTS, vol. 102, 1985, no. 185110b, Columbus, Ohio, US; & JP - A - 59 216 879 (SHIKOKU CHEMICALS CORP.) 06-12-1984

(73) Proprietor: SHIKOKU CHEMICALS CORPORATION
147-1-Minato-machi Marugame-shi
Kagawa-ken(JP)

(72) Inventor: Sawa, Natsuo
5-27, Wakaba-cho Tadotsu-cho
Nakatado-gun Kagawa-ken(JP)
Inventor: Masuda, Takeshi
2-8-15, Kitahirayama-cho
Marugame-shi Kagawa-ken(JP)

(74) Representative: Cresswell, Thomas Anthony et al
J.A. Kemp & Co. 14 South Square Gray's Inn
London WC1R 5EU(GB)



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a process for synthesizing 2-vinyl-4,6-diamino-s-triazine (to be abbreviated as VT).

VT is a compound represented by the following structural formula, and has vinyl-polymerizability.

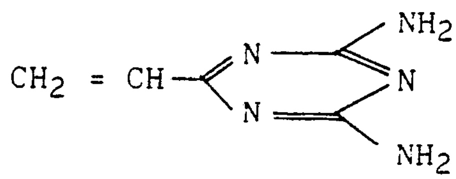

(VT)

VT has a melting point of 239 to 241°C, and is soluble in hot water but sparingly soluble in hot methanol, hot ethanol and hot acetone. It is almost neutral. When VT is dissolved in hot water and azobisisobutyronitrile is added, a polymer insoluble in hot water is formed.

TLC (alumina and silica, EtOH) : Rf = 0.0

$\nu^{KBr}_{cm^{-1}}$ :3340, 3170, 1680 (4th absorption), 1655 (2nd absorption), 1550 (1st absorption), 1460 (5th absorption), 1425 (3rd absorption), 1370, 1265, 1130, 985, 960, 835 (6th absorption).

NMR ($d_6$-DMSO) : $\delta$ 6.76 (m, 4H), 6.35-6.45 (t, 2H), 5.59-5.72 (q, 1H).

Elementary analysis : C 44.28%, H 5.07%, N 50.02%.

VT is useful as a comonomer. It is known that when diamino-s-triazine-group is introduced into a side chain of a polymer, the softening point and glass transition point of the polymer become much higher than the original polymer, its specific gravity also increases, and its solubility changes remarkably (see, for example, Seo and Kakurai: Collection of Papers on Polymers (the Japanese-language publication), 32, 308 (1975); T. Seo, K. Abe, H. Honma, T. Kakurai: Polym. Prepn., 20, 661 (1979)).

Various methods are already known for the synthesis of VT. They include, for example, the reaction of biguanide with acryloyl chloride (C. G. Overberger et al.: J. A. C. S., 80, 988 (1958)), the reaction of dicyandiamide with $\beta$-dimethylamino-propionitrile (Hoechst, French Patent 1,563,255 (1967)), the heating of 1,2-di(4',6'-diamino-s-striazinyl-(2)')-cyclobutane to 320°C under reduced pressure (Asahi Chemical Industry, Co., Ltd.: Japanese Patent Publication NO. 35068/1971), and the heating of 2-$\beta$-methoxyethyl-4,6-diamino-s-triazine to 350°C in a stream of nitrogen (Suddeutsche Kalkstickstoff Werke A.G.: German OLS 2,135,881 (1973)).

JP-A-19772/85 (US-A-4579946) describes a process for producing VT from a betaine. The intermediate betaine has to be produced by reaction of 2MA and acrylic acid and purified before use.

JP-A-216879/84 describes production of VT under reduced pressure from 2MA in the presence of a polymerisation inhibitor, the product being collected as an impure sublimate requiring further purification.

None of these prior methods for the synthesis of VT, however, lends itself to industrial-scale practice for one or more reasons. For example, the starting materials are expensive, the reaction operation is complex, or the yield of VT is low.

The present inventors have now found that VT can be obtained in a high yield by decomposition of 2-(2'-methylimidazole-1'-yl)ethyl-4,6-diamino-s-triazine by heat characterised in that the reaction is conducted in an aqueous medium in the presence of epichlorchydrin and in the presence of a catalytic amount of a polymerisation inhibitor and further characterised in that
2-vinyl-4,6-diamino-s-triazine is recovered as crystals precipitated from the reaction mixture.

This reaction is schematicaly shown as follows:-

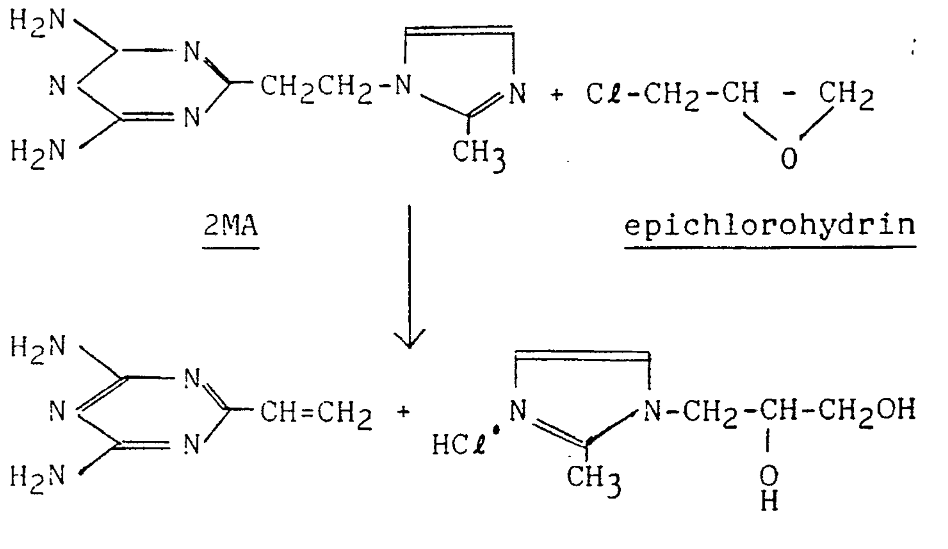

2MA

epichlorohydrin

VT

1-(ß,γ-dihydroxy)-propyl-2-methylimidazole hydrochloride

The starting 2MA in the above reaction is a compound which can be obtained in good yields from acrylonitrile, 2-methylimidazole and dicyandiamide by the method described in Japanese Patent Publication No. 36391/1972.

The other starting material, epichlorohydrin, is a compound which is produced in quantities as a material for general-purpose epoxy resins.

In one preferred embodiment, a starting mixture composed of 1 mole of 2MA, 1 mole of epichlorohydrin, a small amount (i.e., a catalytic amount) of a polymerization inhibitor such as $Na_2S$ $9H_20$ and a suitable amount of water is heated under reflux for 30 minutes with stirring (when heated, the mixture becomes a complete solution, and then crystals begin to precipitate from it).

The resulting reaction mixture is cooled, and the crude product (VT) is collected by filtration. Recrystallization of the crude product in a customary manner gives the final desired product in pure form.

Various commercial polymerization inhibitors, such as hydroquinone and sodium sulfide ($Na_2S \cdot 9H_20$), can be used, but the use of sodium sulfide is most economical.

EXAMPLE

A starting mixture composed of 0.1 mole (21.9 g) of 2MA, 0.1 mole (9.25 g) of epichlorohydrin, a catalytic amount of sodium sulfide ($Na_2S \cdot 9H_20$) and 50 mℓ of water was heated under reflux for 30 minutes with stirring. The resulting reaction mixture was cooled, and 0.1 mole (13.7 g; the yield was quantitative) of the crude product (VT) was collected by filtration. Since the crude product showed a melting point of 237 to 240° C which is much the same as the melting point of an authentic sample of VT, it was considered to be VT having a considerably high purity.

The crude product was recrystallized from water containing a small amount of sodium sulfide to give 0.076 mole (10.4 g; yield based on 2MA 76 mole%) of the purified product. The product had a melting point of 239 to 241° C.

The filtrate left after the recovery of the crude product was neutralized with sodium carbonate. The sodium ion in the neutralized liquid was removed by a sulfonic acid-type ion exchange resin, and then it was concentrated under reduced pressure. The residue was chromatographed on a silica column to give 0.06 mole (9.4 g; yield based on 2MA 60 mole%) of 1-(β,γ-dihydroxy)propyl-2-methylimidazole.

1-(β,γ-Dihydroxy)propyl-2-methylimidazole had the following properties.

Melting point: > 250° C

Colorless and resinous. Basic.

$\nu^{KBr}_{cm^{-1}}$ :1620 (6th absorption), 1585 (3rd absorption), 1520 (2nd absorption), 1415 (2nd absorption), 1255 (2nd absorption), 1100 (1st absorption), 1025 (5th absorption), 860 (5th absorption), 750 (4th absorption).

NMR ($D_2O$) : δ 7.50 (s, 2H(protons at the 4- and 5-positions of imidazole)), 4.34 (m, 5H (propyl group)), 2.72 (s, 3H (methyl group)).

## Claims

1. A process for producing 2-vinyl-4,6-diamino-s-triazine by decomposition of 2-(2'-methylimidazole-1'-yl)-ethyl-4,6-diamino-s-triazine by heat characterised in that the reaction is conducted in an aqueous medium in the presence of epichlorohydrin and in the presence of a catalytic amount of a polymerisation inhibitor and further characterised in that 2-vinyl-4,6-diamino-s-triazine is recovered as crystals precipitated from the reaction mixture.

2. A process according to claim 1, wherein the 2-(2'-methylimidazole-1'-yl)-ethyl-4,6-diamino-s-triazine and epichlorohydrin are reacted in substantially equimolar proportions.

3. A process according to claim 2 wherein the polymerisation inhibitor is sodium sulphide.

4. A process according to any one of claims 1 to 3 wherein the reaction is conducted by heating under reflux.

## Revendications

1. Procédé pour la préparation de 2-vinyl-4,6-diamino-s-triazine par décomposition à la chaleur de 2-(2'-méthylimidazole-1'-yl) éthyl-4,6-diamino-s-triazine caractérisé par le fait que la réaction est conduite en milieu aqueux en présence d'épichlorhydrine et en présence d'une quantité catalytique d'un inhibiteur de polymérisation, et est caractérisé par ailleurs en ce que la 2-vinyl-4,6-diamino-s-triazine est obtenue sous forme de cristaux précipités du milieu réactionnel.

2. Procédé selon la revendication 1, dans lequel la 2-(2'-méthylimidazole-1'-yl)-éthyl-4,6-diamino-s-triazine et l'épichlorhydrine sont mis à réagir en des proportions essentiellement équimolaires.

3. Procédé selon la revendication 2, dans lequel l'inhibiteur de polymérisation est le sulfure de sodium.

4. Procédé selon l'une quelconque des revendications 1 à 3, dans lequel la réaction est conduite par chauffage sous reflux.

## Patentansprüche

1. Verfahren zur Herstellung von 2-Vinyl-4,6-diamino-s-triazin durch Zersetzung von 2-(2'-Methylimidazol-1'-yl)-etyhl-4,6-diamino-s-triazin in der Wärme, dadurch gekennzeichnet, daß die Reaktion in einem wässrigen Medium in Gegenwart von Epichlorhydrin und in Gegenwart einer katalytischen Menge eines Polymerisationsinhibitors ausgeführt wird und weiterhin dadurch gekennezeichnet, daß 2-Vinyl-4,6-diamino-s-triazin als aus dem Reaktionsgemisch ausgefällte Kristalle gewonnen wird.

2. Verfahren nach Anspruch 1, wobei 2-(2'-Methylimidazol-1'-yl)-ethyl-4,6-diamino-s-triazin und Epichlorhydrin in im wesentlichen äquimolaren Anteilen umgesetzt werden.

3. Verfahren nach Anspruch 2, wobei der Polymerisationsinhibitor Natriumsulfid ist.

4. Verfahren nach einem der Ansprüche 1 bis 3, wobei die Reaktion durch Erhitzen unter dem Rückflusskühler durchgeführt wird.